# FASCICULE DE BREVET EUROPEEN

(11) **EP 1 815 806 B1**
(45) Date de publication et mention de la délivrance du brevet: **26.09.2012**
(21) Numéro de dépôt: 06290201.0
(22) Date de dépôt: 06.02.2006
(51) Int. Cl.: A61B 17/17

(54) **Procédé et système pour realiser au moins deux percées traversantes dans un corps oblong**
Verfahren und Vorrichtung, um mindestens zwei Durchgangsbohrungen in einem länglichen Körper durchzuführen
Method and device to implement at least two transverse holes in an oblong body

(43) Date de publication de la demande: 08.08.2007
(73) Titulaire: Bellier, Guy, 75017 Paris (FR); Christel, Pascal, Riyadh 11517 (SA); Colombet, Philippe, 33700 Mérignac (FR); Djian, Patrick, 75017 Paris (FR); Franceschi, Jean-Pierre, 13008 Marseille (FR); Sbihi, Abdou, 13012 Marseille (FR)
(72) Inventeur: Bellier, Guy, 75017 Paris (FR); Christel, Pascal, Riyadh 11517 (SA); Colombet, Philippe, 33700 Mérignac (FR); Djian, Patrick, 75017 Paris (FR); Franceschi, Jean-Pierre, 13008 Marseille (FR); Sbihi, Abdou, 13012 Marseille (FR)
(74) Mandataire: Ouzman, Beverley Nicola Claire

(56) Documents cités:
- FR-A- 2 716 364
- FR-A- 2 727 852
- US-A- 5 688 284

## Description

La présente invention concerne les procédés et les systèmes pour réaliser au moins deux percées traversantes dans un corps oblong comportant une face d'extrémité et une face latérale bordée par la face d'extrémité, et lorsque ces deux percées traversantes doivent déboucher d'une part sur la face d'extrémité respectivement par deux premier et deuxième orifices dont les centres sont distants d'une valeur déterminée A supérieure à la somme des rayons de ces deux orifices et d'autre part sur la face latérale respectivement par deux troisième et quatrième orifices, et ont des axes non confondus faisant entre eux un angle d'une valeur donnée α, en précisant que cette valeur donnée peut être nulle ou non, ce corps oblong pouvant être de toute nature et en tout matériau et ces procédés et dispositifs pouvant trouver des applications dans tous domaines.

Ces percées peuvent par exemple être utilisées, mais non exclusivement, pour passer des liens souples de toute nature en vue de relier le corps les comportant avec un autre.

Il est connu, notamment par les FR-A-2 727 852 et 2 716 364 et le US-A-5 688 284, des systèmes qui permettent de réaliser une percée traversante dans un corps oblong, mais ils ne décrivent pas un procédé qui permet de réaliser de façon cohérente entre elles, deux percées traversantes dans un seul et même corps oblong qui doivent avoir les caractéristiques définies au paragraphe ci-dessus.

La présente invention a pour but de mettre en oeuvre de façon simple un procédé pour réaliser au moins deux percées traversantes dans un corps oblong comportant une face d'extrémité et une face latérale bordée par la face d'extrémité, et lorsque ces deux percées traversantes doivent déboucher d'une part sur la face d'extrémité respectivement par deux premier et deuxième orifices dont les centres sont distants d'une valeur déterminée A supérieure à la somme des rayons de ces deux orifices et d'autre part sur la face latérale respectivement par deux troisième et quatrième orifices, et ont des axes non confondus faisant entre eux un angle d'une valeur donnée α.

La présente invention a aussi pour but de réaliser un système permettant de mettre en oeuvre le procédé selon l'invention.

Plus précisément, la présente invention a pour objet un procédé selon la revendication 1 annexée.

La présente invention a aussi pour objet un système permettant de mettre en oeuvre le procédé défini ci-dessus, selon la revendication 3 annexée.

D'autres caractéristiques et avantages de l'invention apparaîtront au cours de la description suivante donnée en regard des dessins annexés à titre illustratif mais nullement limitatif, dans lesquels :
La figure 1 représente une vue schématique permettant d'expliciter le procédé selon l'invention,
Les figures 2 et 3 représentent un système en deux parties séparées permettant de réaliser des percées. Un mode de réalisation du système selon l'invention réside dans la combinaison des deux parties des figures 2 et 3.

Il est tout d'abord précisé que, sur les figures, les mêmes références désignent les mêmes éléments, quelle que soit la figure sur laquelle elles apparaissent et quelle que soit la forme de représentation de ces éléments. De même, si des éléments ne sont pas spécifiquement référencés sur l'une des figures, leurs références peuvent être aisément retrouvées en se reportant à une autre figure.

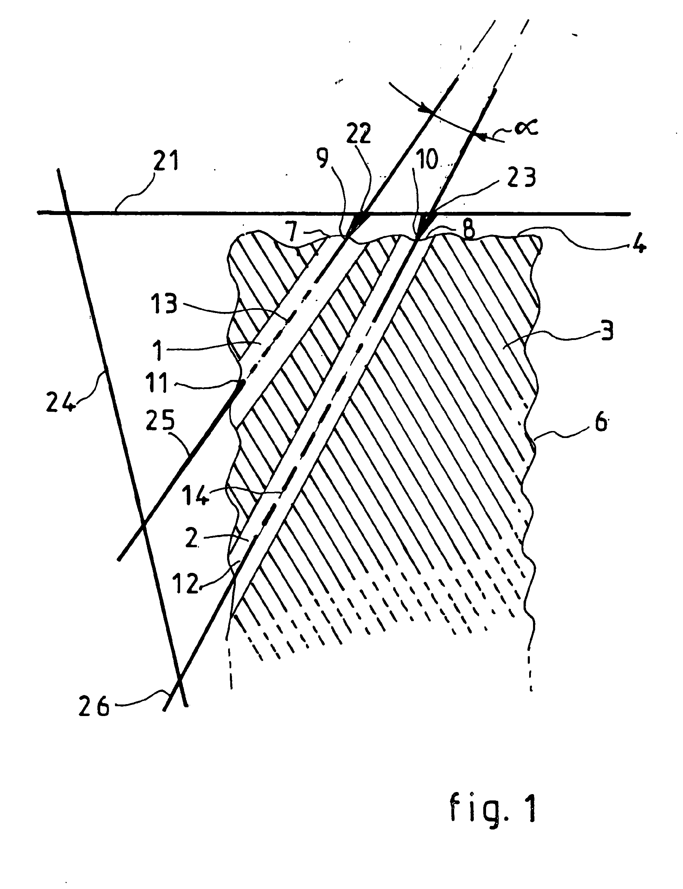

En se référant à la figure 1, le procédé permet de réaliser deux percées traversantes 1, 2 dans un corps oblong 3 comportant une face d'extrémité 4 et une face latérale 6 bordée par la face d'extrémité 4, lorsque ces deux percées traversantes doivent déboucher d'une part sur la face d'extrémité 4 respectivement par deux premier et deuxième orifices 7, 8 dont les centres 9, 10 sont distants d'une valeur déterminée A supérieure à la somme des rayons de ces deux orifices 7, 8 et d'autre part sur la face latérale 6 respectivement par deux troisième et quatrième orifices 11, 12, et ont des axes 13, 14 non confondus faisant entre eux un angle d'une valeur donnée α.

Le procédé selon l'invention consiste à déterminer une première direction 21 définie par deux premier et second points 22, 23 déterminés en relation avec les deux centres 9, 10 des deux premier et deuxième orifices 7, 8, à déterminer une ligne de référence 24 par rapport à la première direction 21, à déterminer une deuxième direction 25 par rapport à la ligne de référence 24 et passant par le premier point 22 de façon que cette deuxième direction 25 soit confondue avec l'axe 13 que doit avoir l'une, la première 1, des deux percées 1, 2, à réaliser la première percée 1 suivant cette deuxième direction 25 à partir de la face latérale 6 à définir une troisième direction 26 par rapport à la deuxième direction (et passant par le second point 23) de façon que les deuxième et troisième directions 25, 26 fassent entre elles un angle de la valeur donnée α, et à réaliser l'autre percée 2 suivant la troisième direction 26.

Dans la description ci-dessus en référence à la figure1, le point 22 est défini comme étant le premier point et le point 23 comme le second, mais il est évident qu'il n'y a dans cette terminologie aucune chronologie obligatoire et que l'un ou l'autre des deux orifices peut être qualifié de "premier" et en conséquence l'autre qualifié de "second".

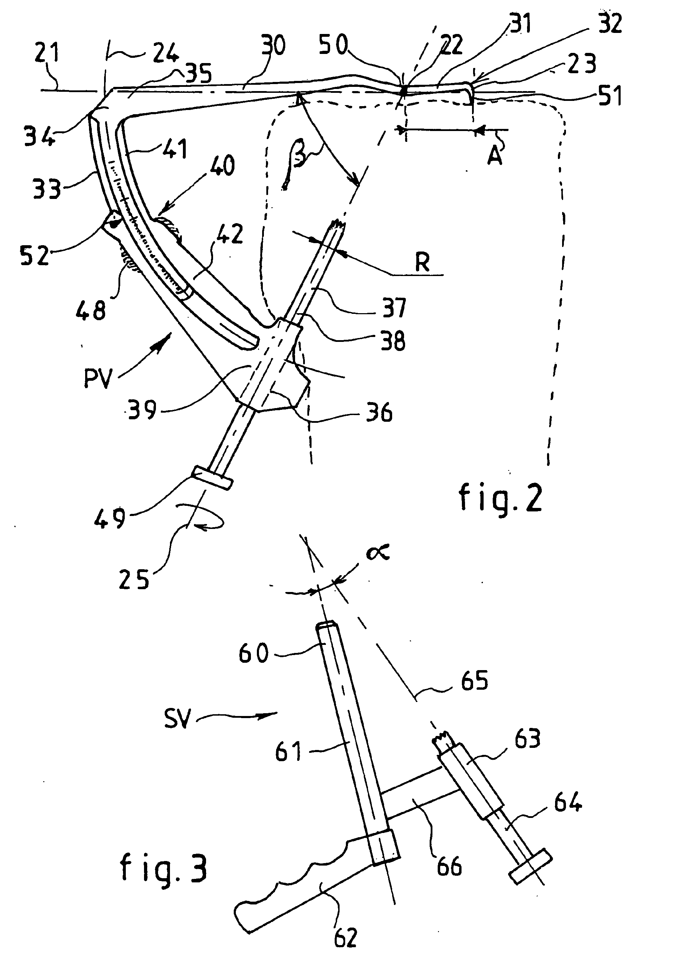

Selon une autre caractéristique avantageuse de l'invention, le procédé consiste à déterminer la ligne de référence 24 suivant une forme circulaire centrée sur l'un des premier et second points, en l'occurrence le premier point 22. Cette caractéristique n'est pas spécifiquement illustrée sur la figure 1, mais apparaît clairement sur la figure 2 qui représente un mode réalisation d'une partie du système selon l'invention permettant de mettre en oeuvre le procédé, partie de système qui sera décrite ci-dessous.

L'invention a en effet aussi pour objet un système permettant de mettre en oeuvre le procédé défini ci-dessus.

Les figures 2 et 3 représentent les deux parties séparées à combiner selon l'invention comportant une patte 30 définie sensiblement sur la première direction 21, cette patte comportant, sensiblement à l'une, première 31, de ses extrémités, des moyens de repérage 32 des premier et second points 22, 23 séparés l'un de l'autre de la distance A, une came de liaison 33 définie sensiblement sur la ligne de référence 24, dont une première extrémité 34 est solidaire de la seconde extrémité 35 de la patte 30, une première tête porte-outil 36, un premier outil de forage 37 monté en rotation dans la première tête porte-outil 36 autour d'un premier axe de forage 38, et des moyens 39 pour monter en coopération la première tête porte-outil 36 avec la came de liaison 33 de façon que le premier axe de forage 38 soit défini sur la deuxième direction 25.

L'outil de forage 37 peut par exemple être constitué par un foret, une fraise ou analogue, qui peut être entraîné par exemple manuellement au moyen d'un bouton moleté 49 (représenté) ou analogue, ou au moyen d'un moteur ou analogue (non représenté).

Selon une caractéristique avantageuse, le premier outil de forage 37 est apte à réaliser des forages de rayon R déterminé par l'homme du métier en fonction du lien ou autre élément à passer dans la percée réalisée.

De façon préférentielle, la came de liaison 33 est constituée d'une glissière 40 comprenant deux première et deuxième pièces complémentaires 41, 42 montées coulissantes l'une par rapport à l'autre suivant la ligne de référence 24, la première pièce 41 (mâle ou femelle) étant solidaire de la patte 30 et la deuxième pièce 42 (femelle ou mâle) solidaire de la première tête porte-outil 36.

De façon avantageuse, comme illustré sur la figure 2, la ligne de référence 24 est définie suivant une courbe circulaire centrée sur le premier point 22 et, dans ce cas, la glissière présente cette forme générale circulaire.

Selon une réalisation possible et avantageuse, les moyens de repérage 32 définis ci-dessus, des premier et second points 22, 23 séparés l'un de l'autre de la distance A sont respectivement constitués par une marque 50 définie sur la patte 30 et un ergot pointu 51 en saillie sur cette patte 30, la distance séparant la marque et l'ergot étant égale A.

Avec cela, il est donc ainsi possible de positionner la patte sur la face 4 du corps 3 en piquant l'ergot pointu 51 dans la face 4 pour définir la position de l'orifice 8 (figure 1) et d'orienter la patte de façon que la marque 50 prenne la position souhaitée que doit prendre l'autre orifice 7 par lequel la première percée 1 doit déboucher sur la face 4.

De même, les deux pièces de glissière complémentaires 41, 42 peuvent être positionnées l'une par rapport à l'autre en fonction de la valeur de l'angle β que doit avoir la deuxième direction 25 par rapport à la première direction 21. Il est donc dans ce cas avantageux que le système comporte des moyens d'indexation 52, par exemple angulaire comme illustré sur la figure 2, des deux pièces complémentaires 41, 42 l'une par rapport à l'autre.

Il est même possible que le système comporte en outre des moyens de blocage 48 des deux pièces complémentaires l'une par rapport à l'autre lorsqu'elles ont leur position respective voulue, par exemple une vis ou analogue.

Lorsque les éléments du premier viseur PV sont positionnés comme décrit ci-dessus et illustré sur la figure 2, la première percée 1 de rayon R peut être réalisée avec l'outil de forage 37 à partir de la face latérale 6 et en débouchant en regard du point 22, par l'orifice 7.

Le système comporte un second viseur SV qui permet de réaliser la seconde percée 2.

Un second viseur, illustré cependant sur la figure 3, que séparément peut comporte un guide 60 défini selon un axe de révolution 61, des moyens 62 pour maintenir le guide, une seconde tête porte-outil 63, un second outil de forage 64 monté en rotation dans la seconde tête porte-outil 63 autour d'un second axe de forage 65, et une entretoise de liaison 66 reliant la seconde tête porte-outil 63 et le guide 60 de façon que les premier et second axes de forage 61, 65 fassent entre eux un angle de valeur α.

Les moyens 62 pour maintenir le guide 60 sont constitués d'une poignée, comme illustré sur la figure 3, et le guide 60 est cylindrique de révolution de rayon égal à R.

Avec ce second viseur, il est possible de réaliser une seconde percée 2. Pour cela le guide 60 est introduit dans la première percée 1 réalisée comme décrit ci-dessus, et y est maintenu par exemple manuellement avec la poignée. Le second outil de forage 64 est alors mis en rotation sur l'axe de forage 65 pour réaliser la seconde percée. L'axe 14 de cette seconde percée 2 fait donc avec l'axe 13 de la première percée 1 un angle de la valeur donnée α.

Cependant, le système selon l'invention est realisée combinant les deux parties PV et SV. Dans ce cas, l'outil de forage 37 de la première partie PV du système est utilisé comme guide 60 pour la seconde partie SV.

Le système permettant de mettre en oeuvre le procédé défini précédemment, comporte alors la patte 30 définie sensiblement sur la première direction 21, cette patte comportant, sensiblement à l'une, première 31, de ses extrémités, les moyens de repérage 32 des premier et second points 22, 23 séparés l'un de l'autre de la distance A, la came de liaison 33 définie sensiblement sur la ligne de référence 24, dont une première extrémité 34 est solidaire de la seconde extrémité 35 de la patte 30, la première tête porte-outil 36, le premier outil de forage 37 monté en rotation dans la première tête porte-outil 36 autour du premier axe de forage 38, les moyens 39 pour monter en coopération la première tête porte-outil 36 avec la came de liaison 33 de façon que le premier axe de forage 38 soit défini sur la deuxième direction 25, la seconde tête porte-outil 63, le second outil de forage 64 monté en rotation dans la seconde tête porte-outil 63 autour du second axe de forage 65, l'entretoise de liaison 66 reliant la seconde tête porte-outil 63 et la première tête porte-outil 36 de façon que les premier et second axes de forage 38, 65 fassent entre eux un angle de valeur α.

Selon cette réalisation, la patte 30 et la glissière 40 sont positionnées comme décrit ci-dessus, la première percée 1 est réalisée comme décrit ci-dessus puis, tout en maintenant le premier outil de forage 37 dans la première percée, la seconde percée 2 est réalisée comme décrit ci-dessus.

Il est aussi précisé que les deux percées 1 et 2 peuvent avoir le même diamètre ou des diamètres différents et que les deux outils de forage 37, 64 sont adaptés à la valeur de ces diamètres.

## Revendications

1. Procédé pour réaliser deux percées traversantes (1, 2) dans un corps oblong (3) comportant une face d'extrémité (4) et une face latérale (6) bordée par la face d'extrémité (4), et lorsque ces deux percées traversantes :
• doivent déboucher d'une part sur la face d'extrémité (4) respectivement par deux premier et deuxième orifices (7, 8) ayant des centres (9, 10) et d'autre part sur la face latérale (6) respectivement par deux troisième et quatrième orifices (11, 12), et
• ont respectivement des axes (13, 14) non confondus et faisant entre eux un angle d'une valeur donnée α, le procédé étant **caractérisé par le fait qu'**il consiste :
- à déterminer une première direction (21) définie par deux premier et second points (22, 23) déterminés en relation avec les deux centres (9, 10) des deux premier et deuxième orifices (7, 8) les centres (9, 10) étant distants d'une valeur déterminée A supérieure à la somme des rayons de ces deux orifices (7, 8)
- à déterminer une ligne de référence (24) par rapport à la première direction (21),
- à déterminer une deuxième direction (25) par rapport à la ligne de référence (24) et passant par le premier point (22) de façon que cette deuxième direction (25) soit confondue avec l'axe (13) que doit avoir l'une (1) des deux percées (1, 2) à réaliser,
- à réaliser la première percée (1) suivant cette deuxième direction (25) à partir de la face latérale (6),
- à définir une troisième direction (26) par rapport à la deuxième direction, de façon que les deuxième et troisième directions (25, 26) fassent entre elles un angle de la valeur donnée α, et
- à réaliser l'autre seconde percée (2) suivant la troisième direction (26) de manière que les deux centres (9, 10) soient distants l'un de l'autre de ladite valeur déterminée A.

2. Procédé selon la revendication 1, **caractérisé par le fait qu'**il consiste à déterminer la ligne de référence (24) suivant une forme circulaire centrée sur le premier point (22), la deuxième direction (25) étant une direction diamétrale de ladite forme circulaire.

3. Système permettant de réaliser deux percées traversantes (1, 2) dans un corps oblong (3) comportant une face d'extrémité (4) et une face latérale (6) bordée par la face d'extrémité (4), et lorsque ces deux percées traversantes doivent déboucher d'une part sur la face d'extrémité (4) respectivement par deux premier et deuxième orifices (7, 8) ayant des centres (9,10) et d'autre part sur la face latérale (6) respectivement par deux troisième et quatrième orifices (11, 12), et ont respectivement des axes (13, 14) non confondus et faisant entre eux un angle d'une valeur donnée α, le système étant
• une patte (30) définie sensiblement sur une premiére direction (21) définie par deux premier et second points (22, 23) déterminés en relation avec les deux centres (9, 10) des deux premier et deuxième orifices (7, 8) à réaliser les centres (9, 10) étant distants d'une valeur déterminée A supérieure à la somme des rayons de ces deux orifices (7, 8) ladite patte comportant, sensiblement à l'une, première (31), de ses extrémités, des moyens de repérage (32) des premier et second points (22, 23) séparés l'un de l'autre de la distance A,
• une came de liaison (33) définie sensiblement sur une ligne de référence (24) définie par rapport à ladite direction (21), dont une première extrémité (34) est solidaire de la seconde extrémité (35) de la patte (30),
• une première tête porte-outil (36),
• un premier outil de forage (37) monté en rotation dans la première tête porte-outil (36) autour d'un premier axe de forage (38), ce dit premier outil (37) étant apte à réaliser la première percée (1),
• des moyens (39) pour monter en coopération la première tête porte-outil (36) avec la came de liaison (33),
• une seconde tête porte-outil (63),
• un second outil de forage (64) monté en rotation dans ladite seconde tête porte-outil (63) autour d'un second axe de forage (65), ce dit second outil étant apte à réaliser ladite seconde percée (2), et
• une entretoise de liaison (66) reliant la seconde tête porte-outil (63) et la première tête porte-outil (36) de façon que les premier et second axes de forage (61, 65) fassent entre eux un angle de valeur α.

4. Système selon la revendication 3, **caractérisé par le fait que** ledit premier outil de forage (37) est apte à réaliser des forages de rayon R.

5. Système selon l'une des revendications 3 et 4, **caractérisé par le fait que** ladite came de liaison (33) est constituée d'une glissière (40) comprenant deux première et deuxième pièces complémentaires (41, 42) montées coulissantes l'une par rapport à l'autre suivant la ligne de référence (24), la première pièce complémentaire de glissière (41) étant solidaire de la patte (30) et la deuxième pièce complémentaire de glissière (42) solidaire de la première tête porte-outil (36).

6. Système selon l'une des revendications 3 à 5, **caractérisé par le fait que** la ligne de référence (24) est définie suivant une courbe circulaire centrée sur le premier point (22).

7. Système selon l'une des revendications 3 à 6, **caractérisé par le fait que** les moyens de repérage (32) des premier et second points (22, 23) séparés l'un de l'autre d'une distance A sont respectivement constitués par une marque (50) définie sur ladite patte (30) et un ergot pointu (51) en saillie sur ladite patte (30), la distance séparant ladite marque et ledit ergot étant égale A.

8. Système selon l'une des revendications 5 à 7, **caractérisé par le fait qu'**il comporte des moyens d'indexation (52) des deux parties de glissière (41, 42) l'une par rapport à l'autre.

## Claims

1. Method of forming two through bores (1, 2) in an oblong body (3) comprising an end face (4) and a side face (6) bordered by the end face (4), and when these two through bores:
• must emerge on the one hand on the end face (4) respectively through two first and second orifices (7, 8) having centres (9, 10) and on the other hand on the side face (6) respectively through two third and fourth orifices (11, 12), and
• respectively have non-coincident axes (13, 14) forming between them an angle of a given value α, the method being **characterised by** the fact that it consists:
- in determining a first direction (21) defined by two first and second points (22, 23) determined relative to the two centres (9, 10) of the two first and second orifices (7, 8), the centres (9, 10) being distant by a determined value A greater than the sum of the radii of these two orifices (7, 8),
- in determining a reference line (24) relative to the first direction (21),
- in determining a second direction (25) relative to the reference line (24) and passing through the first point (22) so that this second direction (25) coincides with the axis (13) which one (1) of the two bores (1, 2) to be formed must have,
- in forming the first bore (1) in this second direction (25) from the side face (6),
- in defining a third direction (26) relative to the second direction, so that the second and third directions (25, 26) form between them an angle of the given value α, and
- forming the other second bore (2) in the third direction (26) in such a manner that the two centres (9, 10) are distant the one from the other by said determined value A.

2. Method according to claim 1, **characterised by** the fact that it consists in determining the reference line (24) in a circular form centred on the first point (22), the second direction (25) being a diametric direction of said circular form.

3. System permitting the formation of two through bores (1, 2) in an oblong body (3) comprising an end face (4) and a side face (6) bordered by the end face (4), and when these two through bores must emerge on the one hand on the end face (4) respectively through two first and second orifices (7, 8) having centres (9, 10) and on the other hand on the side face (6) respectively through two third and fourth orifices (11, 12), and respectively have non-coincident axes (13, 14) forming between them an angle of a given value α, the system being **characterised by** the fact that it comprises:
• a leg (30) substantially defined in a direction (21) defined by two first and second points (22, 23) determined relative to the two centres (9, 10) of the two first and second orifices (7, 8) to be formed, the centres (9, 10) being distant by a determined value A greater than the sum of the radii of these two orifices (7, 8), said leg comprising, substantially at one, first (31), of its ends, means for locating (32) the first and second points (22, 23) separated the one from the other by the distance A,
• a connecting cam (33) defined substantially on one reference line (24) defined relative to said direction (21), a first end (34) of which is attached to the second end (35) of the leg (30),
• a first tool-carrying head (36),
• a first drilling tool (37) mounted in rotation in the first tool-carrying head (36) about a first drilling axis (38), this first tool (37) being able to form the first bore (1),
• means (39) for assembling in cooperation the first tool-carrying head (36) with the connecting cam (33), (61),
• a second tool-carrying head (63),
• a second drilling tool (64) mounted in rotation in said second tool-carrying head (63) about a second drilling axis (65), this said second tool being able to form said second bore (2), and
• a connecting strut (66) joining the second tool-carrying head (63) and the first tool-carrying head (36) so that the first and second drilling axes (61, 65) form between them an angle of value α.

4. System according to claim 3, **characterised by** the fact that said first drilling tool (37) is able to form bores of radius R.

5. System according to one of claims 3 and 4, **characterised by** the fact that said connecting cam (33) is formed of a slide (40) including two first and second complementary pieces (41, 42) mounted slidingly the one relative to the other along the reference line (24), the first complementary slide piece (41) being attached to the leg (30) and the second complementary slide piece (42) attached to the first tool-carrying head (36).

6. System according to one of claims 3 to 5, **characterised by** the fact that the line of reference (24) is defined in a circular curve centred on the first point (22).

7. System according to one of claims 3 to 6, **characterised by** the fact that the means for locating (32) the first and second points (22, 23) separated the one from the other by a distance A are respectively formed by a mark (50) defined on said leg (30) and a pointed lug (51) projecting on said leg (30), the distance separating said mark and said lug being equal to A.

8. System according to one of claims 5 to 7, **characterised by** the fact that it comprises means for indexing (52) the two slide parts (41, 42) the one relative to the other.

## Patentansprüche

1. Ein Verfahren zum Fertigen von zwei Durchgangsbohrungen (1, 2) in einem länglichen Körper (3), der eine Endfläche (4) und eine Seitenfläche (6), die von der Endfläche (4) begrenzt wird, beinhaltet und wobei diese zwei Durchgangsbohrungen:
• einerseits durch zwei Öffnungen (7, 8), eine erste beziehungsweise eine zweite, die Zentren (9, 10) aufweisen, zu der Endfläche (4) und andererseits jeweils durch zwei Öffnungen (11, 12), eine dritte beziehungsweise eine vierte, zu der Seitenfläche (6) hinführen müssen, und
• jeweils nicht übereinstimmende Achsen (13, 14) aufweisen und zwischen sich einen Winkel eines gegebenen Werts α bilden, wobei das Verfahren **dadurch gekennzeichnet ist, dass** es aus Folgendem besteht:
- Festlegen einer ersten Richtung (21), die durch zwei Punkte (22, 23), einen ersten und einen zweiten, definiert ist, die mit Bezug auf die zwei Zentren (9, 10) der zwei Öffnungen (7, 8), der ersten und der zweiten, festgelegt sind, wobei die Zentren (9, 10) um einen festgelegten Wert A, der größer als die Summe der Radien der zwei Öffnungen (7, 8) ist, voneinander beabstandet sind,
- Festlegen einer Bezugslinie (24) bezogen auf die erste Richtung (21),
- Festlegen einer zweiten Richtung (25) bezogen auf die Bezugslinie (24) und die durch den ersten Punkt (22) läuft, so dass diese zweite Richtung (25) mit der Achse (13), die die eine (1) der zwei zu fertigenden Bohrungen (1, 2) aufweisen muss, übereinstimmt,
- Fertigen der ersten Bohrung (1) gemäß dieser zweiten Richtung (25) von der Seitenfläche (6) aus,
- Definieren einer dritten Richtung (26) bezogen auf die zweite Richtung, so dass die zweite und die dritte Richtung (25, 26) zwischen sich einen Winkel mit dem gegebenen Wert α bilden, und
- Fertigen der anderen, zweiten Bohrung (2) gemäß der dritten Richtung (26), so dass die zwei Zentren (9, 10) in dem festgelegten Wert A voneinander entfernt liegen.

2. Verfahren gemäß Anspruch 1, **dadurch gekennzeichnet, dass** es aus dem Festlegen der Bezugslinie (24) gemäß einer Kreisform, die auf dem ersten Punkt (22) zentriert ist, besteht, wobei die zweite Richtung (25) eine diametrale Richtung der Kreisform ist.

3. Ein System, das das Fertigen von zwei Durchgangsbohrungen (1, 2) in einem länglichen Körper (3), der eine Endfläche (4) und eine Seitenfläche (6), die von der Endfläche (4) begrenzt wird, beinhaltet, ermöglicht, und wobei diese zwei Durchgangsbohrungen einerseits durch zwei Öffnungen (7, 8), eine erste beziehungsweise eine zweite, die Zentren (9, 10) aufweisen, zu der Endfläche (4) und andererseits jeweils durch zwei Öffnungen (11, 12), eine dritte beziehungsweise vierte, zu der Seitenfläche (6) hinführen müssen und jeweils nicht übereinstimmende Achsen (13, 14) aufweisen und zwischen sich einen Winkel eines gegebenen Werts α bilden, wobei das System **dadurch gekennzeichnet ist, dass** es Folgendes beinhaltet:
• einen Schenkel (30), der im Wesentlichen nach einer ersten Richtung (21) definiert ist, die durch zwei Punkte (22, 23), einen ersten und einen zweiten, definiert ist, die mit Bezug auf die zwei Zentren (9, 10) der zwei zu fertigenden Öffnungen (7, 8), einer ersten und einer zweiten, festgelegt sind, wobei die Zentren (9, 10) um einen festgelegten Wert A, der größer als die Summe der Radien dieser zwei Öffnungen (7, 8) ist, voneinander beabstandet sind, wobei der Schenkel im Wesentlichen an einem ersten (31) seiner Enden Markierungsmittel (32) eines ersten und zweiten Punkts (22, 23), die voneinander durch die Entfernung A getrennt sind, beinhaltet,
• eine Verbindungsnocke (33), die im Wesentlichen auf einer Bezugslinie (24) definiert ist, die bezogen auf die Richtung (21) definiert ist, bei der ein erstes Ende (34) formschlüssig mit dem zweiten Ende (35) des Schenkels (30) verbunden ist,
• einen ersten Werkzeugträgerkopf (36),
• ein erstes Bohrwerkzeug (37), das in dem ersten Werkzeugträgerkopf (36) um eine erste Bohrachse (38) drehbar montiert ist, wobei dieses erste Werkzeug (37) geeignet ist, um die erste Bohrung (1) zu fertigen,
• Mittel (39) zum Montieren des ersten Werkzeugträgerkopfs (36) in Zusammenarbeit mit der Verbindungsnocke (33), (61),
• einen zweiten Werkzeugträgerkopf (63),
• ein zweites Bohrwerkzeug (64), das in dem zweiten Werkzeugträgerkopf (63) um eine zweite Bohrachse (65) drehbar montiert ist, wobei dieses zweite Werkzeug geeignet ist, um die zweite Bohrung (2) zu fertigen, und
• einen Verbindungssteg (66), der den zweiten Werkzeugträgerkopf (63) und den ersten Werkzeugträgerkopf (36) verbindet, so dass die erste und die zweite Bohrachse (61, 65) zwischen sich einen Winkel mit dem Wert α bilden.

4. System gemäß Anspruch 3, **dadurch gekennzeichnet, dass** das erste Bohrwerkzeug (37) geeignet ist, um Bohrungen mit Radius R zu fertigen.

5. System gemäß einem der Ansprüche 3 und 4, **dadurch gekennzeichnet, dass** die Verbindungsnocke (33) aus einer Gleitschiene (40) zusammengesetzt ist, die zwei Komplementärstücke (41, 42), ein erstes und ein zweites, die bezogen aufeinander gemäß der Bezugslinie (24) verschiebbar montiert sind, beinhaltet, wobei das erste Gleitschienenkomplementärstück (41) mit dem Schenkel (30) formschlüssig verbunden ist und das zweite Gleitschienenkomplementärstück (42) mit dem ersten Werkzeugträgerkopf (36) formschlüssig verbunden ist.

6. System gemäß einem der Ansprüche 3 bis 5, **dadurch gekennzeichnet, dass** die Bezugslinie (24) gemäß einer kreisförmigen Krümmung, die auf dem ersten Punkt (22) zentriert ist, definiert ist.

7. System gemäß einem der Ansprüche 3 bis 6, **dadurch gekennzeichnet, dass** die Markierungsmittel (32) des ersten und des zweiten Punkts (22, 23), die durch eine Entfernung A voneinander getrennt sind, aus einer Markierung (50), die auf dem Schenkel (30) definiert ist, beziehungsweise einem spitzen Sporn (51), der auf dem Schenkel (30) vorstehend ist, zusammengesetzt sind, wobei die Entfernung, die die Markierung und den Sporn trennt, gleich A ist.

8. System gemäß einem der Ansprüche 5 bis 7, **dadurch gekennzeichnet, dass** es Positionierungsmittel (52) der zwei Gleitschienenteile (41, 42) bezogen aufeinander beinhaltet.
